# EUROPEAN PATENT SPECIFICATION

(11) **EP 4 504 474 B1**
(45) Date of publication and mention of the grant of the patent: **10.09.2025**
(21) Application number: 23722941.4
(22) Date of filing: 04.04.2023
(51) Int. Cl.: B28B 11/24, B28B 17/00, B28B 3/02, B29C 67/24

(54) **CATALYSIS OVEN AND METHOD FOR CONTROLLING THE OPERATION OF A CATALYSIS OVEN**
KATALYSEOFEN UND VERFAHREN ZUR STEUERUNG DES BETRIEBS EINES KATALYSEOFENS
FOUR DE CATALYSE ET PROCÉDÉ DE COMMANDE DU FONCTIONNEMENT D'UN FOUR DE CATALYSE

(30) Priority: 06.04.2022 IT 202200006776
(43) Date of publication of application: 12.02.2025
(73) Proprietor: Toncelli, Dario, 36061 Bassano del Grappa (VI) (IT)
(72) Inventor: Toncelli, Dario, 36061 Bassano del Grappa (VI) (IT)
(74) Representative: Dragotti & Associati S.R.L.
(86) International application number: PCT/IB2023/053385
(87) International publication number: WO 2023/194887

(56) References cited:
- WO-A1-2010/024220
- WO-A1-2017/010984
- DE-A1- 3 914 106
- JP-A- 2021 122 840
- US-A- 4 937 024
- US-A1- 2002 014 710
- US-B2- 6 983 684

## Description

The present invention relates to the technical field of the manufacture of slabs of agglomerated stone or stone-like material from a mix containing granules and/or aggregates of stone or stone-like material and a binder, preferably an organic binder or an organic resin. In particular, the present invention relates to an oven intended to perform the catalysis of the binder and hardening of the mix by means of heating in order to form the slabs.

The mix is subjected to at least one compaction step, preferably by means of vacuum vibro-compression, performed before the heating and hardening step.

The present invention also relates to a method for controlling the operation of the aforementioned catalysis oven.

Ovens designed to perform a step involving heating and hardening of a mix in order to form slabs of agglomerated material by means of a catalysis reaction of the binder, or the resin, contained in the mix, are known.

Such a catalysis and hardening step carried out by means of the oven is performed following a step involving distribution of the mix on a temporary support and following a step of compaction of the mix, preferably by means of vacuum vibro-compression.

The mix distributed in the temporary support is intended generally to be covered with a containing element or cover which forms a sheath or casing with the temporary support; the compaction step, preferably performed by means of vacuum vibro-compression, is performed on the mix contained inside the casing.

The process which involves the steps of distribution, vacuum vibro-compression and hardening of the mix is also known as the *Bretonstone*^{®} process.

Furthermore, this process preferably comprises a step of cooling the slabs carried out following the catalysis and hardening step.

The catalysis and hardening step is performed by heating the mix by means of application a predetermined quantity of heat, said quantity of heat depending in particular on the quantity of binder contained in the mix and the dimensions and the thickness of the slab to be manufactured.

A part of the quantity of heat supplied is partially absorbed also by the casing containing the mix.

For example, for a slab enclosed in a rubberized cloth casing with sizes of 330 cm x 165 cm, a thickness of 35 mm and weight amount of unsaturated polyester resin equal to 10% of the overall weight of the mix, the heat required to perform the catalysis step is equal to about 15,400 kcal, 3,700 kcal of which are absorbed by the casing, namely by the support and the cover.

Usually, the duration of the catalysis step for slabs with a thickness close to 35 mm is equal to about 30 minutes; during this time interval, the mix must reach a threshold temperature, also called flash point temperature, close to 75-80°C, so that the catalysis reaction may be triggered and then maintained.

Moreover, it should be considered that the catalysis reaction of a polyester resin is an exothermic reaction which generates heat; therefore the part played by the catalysis oven in the administration of the quantity of heat indicated above may also be limited.

The known catalysis ovens have a vertically extending frame comprising a series of pairs of heating surfaces; the surfaces of each pair delimit a housing for positioning a respective casing inside which the mix is distributed and compacted.

Moreover, for each pair a first surface, or bottom surface, and a second surface, or top surface, are defined. In the context of the present description "bottom surface" is understood as meaning the surface of each pair situated at a smaller distance from the ground.

Usually, the bottom surfaces are positioned at a set height or at a variable height, and the top surfaces, associated with the corresponding bottom surfaces, are movable with respect to the bottom surfaces so as to vary the dimensions of the housings and allow the insertion of the casings with the compacted mix.

Each casing positioned inside the respective housing is supported by the bottom heating surface; after positioning of the slab, the top heating surface is moved so as to rest on the slab and uniformly heat the mix; the transmission of heat usually occurs essentially by means of heat conduction.

Alternatively, the transmission of the heat between the heating surfaces and the casings with the mix may also take place by means of irradiation.

The top surfaces may also be positioned at a few millimetres distance from the surface of the slab; in this case, heating from above takes place substantially by means of irradiation, while heating from below occurs in any case by means of conduction, since the casing with the mix rests on the bottom surface.

The quantity of heat transmitted from the heating surfaces to the casings containing the mix and necessary for starting the catalysis reaction depends mainly on the temperature at which the heating surfaces are kept and the time for which the casing is kept inside the respective housing.

The surfaces are preferably made of aluminium so as to have a relatively low specific weight and high thermal conductivity.

Furthermore, the heating surfaces usually comprise respective internal circuits, preferably coils, for conveying a heating fluid kept at a relatively high temperature (generally close to 90-130°C); the heating fluid is composed preferably of diathermic oil.

The heating fluid, or diathermic oil, is supplied to the heating surfaces by means of at least one primary supply circuit connected to at least one exchanger of a boiler which operates normally at temperatures close to 200°C.

Furthermore, the heating fluid is supplied to the heating surfaces by means of one or more secondary supply circuits which normally operate at temperatures close to 90-130°C and are connected to the internal circuits of the heating surfaces and to the primary circuit.

The known catalysis ovens also comprise one or more electric pumps positioned in the primary circuit and/or in the secondary circuits for supplying the heating fluid inside these circuits.

The primary supply circuit may comprise one or more branches connected to a respective secondary circuit.

Each branch comprises a delivery section for supplying the fluid to the respective secondary circuit and a return section which receives the heating fluid from the said secondary circuit after supplying the internal circuits of the heating surfaces.

The known catalysis ovens may also comprise a series of pneumatic three-way valves arranged in the primary circuit, preferably in the various branches of the primary circuit and even more preferably between each delivery section and each return section.

These three-way valves allow regulation of the flow of the heating fluid to the primary circuit and consequently regulate the amount of heat released to the secondary circuits and therefore also the temperature of the heating surfaces.

For example, if the temperature of the surfaces must be reduced, the three-way valve is regulated so as to deviate a greater quantity of heating fluid towards the return section of the primary circuit instead of towards the secondary circuits.

Furthermore, the known catalysis ovens generally have a first secondary circuit for supplying the heating fluid to the top surfaces of each pair and a second secondary circuit for supplying the heating fluid to the bottom surfaces of each pair.

This arrangement allows the temperature of the top heating surfaces to be regulated independently of the temperature regulation of the bottom heating surfaces.

An undesirable reaction of the technical solutions described above is associated mainly with the possible non-uniform volumetric shrinkage of the binder contained in the mix during the catalysis step and on the opposite faces of the slab to be hardened; this reaction results in the formation of curvatures, deformations or internal tension in the slab.

The cause of any non-uniform volumetric shrinkage lies generally in a different heat flow on the two faces of the slab. The different heat flow may be due to the fact that the heating surfaces, in particular the top surfaces, may not make perfect contact with the mix inside the casing during the catalysis and hardening step or to the fact that the surfaces of the slab and/or of the casing may be slightly undulating or in any case not perfectly flat.

In this way, air bubbles or pockets may form between the surfaces and the casings containing the mix; these air bubbles or pockets complicate in particular control of the heat energy flow from the surfaces of the slab during the entire process of catalysis and hardening of the mix, since they limit, or result in the irregular transmission of, the heat from the surfaces to the casing.

In order to overcome at least partially the aforementioned drawback, the heating fluid supplied to the top surfaces, where the formation of bubbles or pockets is usually concentrated, may have a temperature which is between 3 and 15°C higher than the temperature of the fluid supplied to the bottom surfaces.

Alternatively, the top surfaces may be kept spaced by a few millimetres from the surface of the slab; this arrangement allows the temperature of the top surfaces to be increased by about 30°C with respect to the bottom surfaces and for heat transmission to be performed by means of irradiation instead of conduction, so as to control more effectively the heat flow.

A further measure which is commonly used in catalysis ovens is that of maintaining a fast flow of heating fluid inside the circuits so that the flow is turbulent and not laminar. A turbulent flow of the fluid ensures a more efficient heat exchange between the fluid and the heating surfaces.

The combination of the technical measures indicated above, in addition to partially overcoming the drawback mentioned above, is also able to prevent or in any case limit the formation of curvatures, deformations or internal tensions in the slabs.

Despite the abovementioned advantages which can be achieved and their wide use in the field, the aforementioned technical solutions are not without a number of drawbacks.

A first drawback consists in the fact that the temperature of each heating surface may not be regulated independently of the other heating surfaces, at least the other surfaces of the same set (top surfaces or bottom surfaces).

As a result of this drawback it not possible to insert simultaneously into the oven slabs which have a different composition and which therefore require different catalysis temperatures.

The operator is therefore obliged to perform the catalysis and hardening step by introducing into the oven mixes with identical or at least similar features, or similar operating conditions, in order to manufacture the slabs.

As a result the known catalysis ovens have particularly limited flexibility in terms of their use.

US 6983684 B2 relates to an apparatus for the production of wood composite boards and discloses the features of the preamble of claim 1.

The main object of the present invention is to provide a catalysis oven for the formation of slabs of agglomerated material and a method for controlling said catalysis oven which are able to overcome the aforementioned drawbacks.

A particular task of the present invention is to provide a catalysis oven of the type indicated above which is able to perform the catalysis and hardening step in an efficient manner also in the case of mixes and slabs which have mutually different features.

A further task of the present invention is to provide a catalysis oven of the type described above which is able to limit the formation of defects, such as curvatures, deformations or internal tensions, in the slabs at the end of the catalysis step.

Another task of the present invention is to provide a catalysis oven of the type described above which has a particularly high flexibility in terms of use.

A further task of the present invention is to provide a catalysis oven of the type described above which ensures constant and uniform operating conditions for the catalysis and hardening of the mixes.

Another task of the present invention is to provide a catalysis oven of the type described above in which there is uniform shrinkage of the slab during the catalysis and hardening step. A further task of the present invention is to provide a catalysis oven of the type described above which has a limited costs and complexity.

Another task of the present invention is to provide a method for controlling a catalysis oven of the type described above which allows predefined operating conditions to be defined for mixes with different characteristics.

The object and the main tasks described above are achieved with a catalysis oven according to Claim 1 and with a method for controlling the operation of the catalysis oven according to Claim 14.

In order to illustrate more clearly the innovative principles of the present invention and its advantages compared to the prior art, an example of embodiment of the catalysis oven according to the present invention will be described below with the aid of the attached figures. In particular, in the figures:
- Figure 1 is a schematic view of the components of the catalysis oven according to the present invention;
- Figure 2 is a schematic block-diagram view of the system for controlling the catalysis oven according to the present invention.

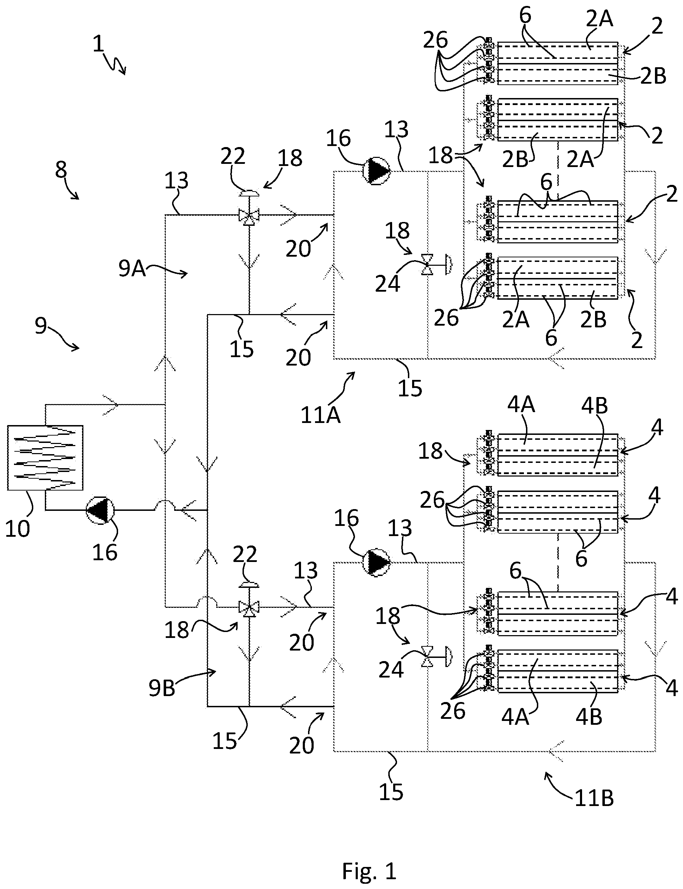

The present description, provided only by way of a non-limiting example of the scope of protection of the invention, relates to a catalysis oven shown schematically in Figure 1 and denoted overall by the reference number 1.

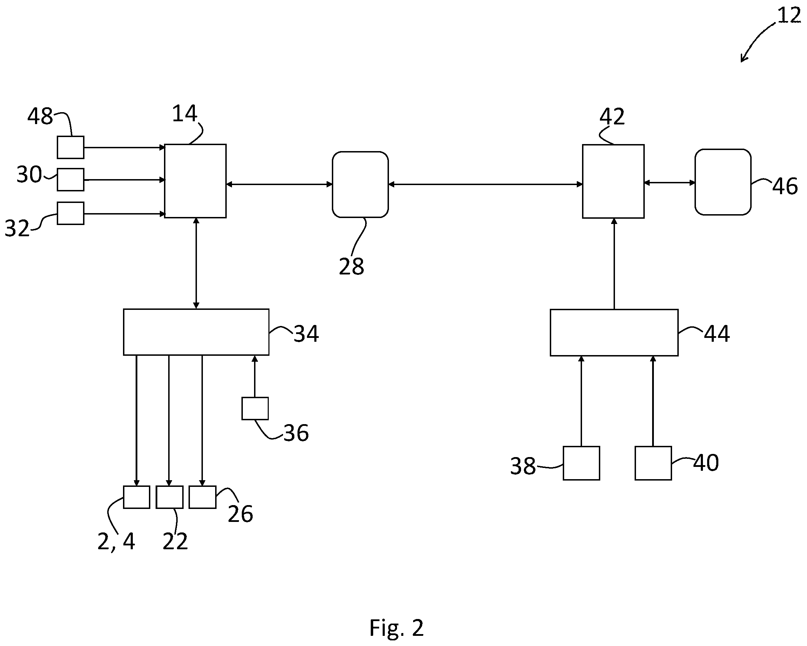

Figure 2 shows in schematic form some of the components of the catalysis oven with reference to the system for control thereof.

In particular, the catalysis oven 1 of the present invention is intended to perform a step of catalysis and hardening of an agglomerated mix of stone or stone-like material by means of heating of the compacted mix contained inside a casing or sheath.

The mix is intended to form slabs of agglomerated stone or stone-like material and is formed by powder, granules and aggregates of stone or stone-like material and by a binder, preferably an organic binder or an organic resin.

The hardening of the mix for formation of the slabs is obtained by means of catalysis of the binder, namely the organic resin, contained inside it.

Advantageously, the heating and hardening step is performed, in accordance with the operating modes described below, by heating the mix to a temperature, depending on the type of resin, which is usually about 90-110°C for a time period close to 30 minutes, with particular reference to a slab to be made with a thickness of about 35 mm.

In addition to the step of heating and hardening the mix, the process for making slabs involves beforehand a step involving distribution of the mix on a temporary support, with subsequent covering of the mix with a containment element or cover, a step involving compaction of the mix, preferably by means of vacuum vibro-compression and, then, a step of cooling of the slabs.

Therefore, the distribution and vibro-compression steps are performed upstream of the heating and hardening step, while the cooling step is performed downstream of the heating step.

The distribution, compaction and cooling steps are performed by means of known devices and apparatus which will not be further described in the continuation of the present description; for example, the cooling step is generally performed in a slab cooling tower.

The catalysis oven of the present invention is included in a plant for the production of slabs from a mix, which also comprises the devices and/or the apparatus mentioned above.

The casings, which are not shown in the attached figures, are formed by the supports and the covers and are made mainly of elastomeric material; furthermore, the supports are preferably of the tray type and have a cavity delimited by a rim portion for containing the mix inside them.

The catalysis oven 1 preferably comprises:
- a series of pairs of heating surfaces 2, 4 which delimit respective housings for the casings/sheaths containing the mix, each of the heating surfaces 2, 4 comprising at least one internal circuit 6 for a heating fluid;
- at least one supply circuit 8 for the heating fluid, connected to the internal circuits 6 of the heating surfaces 2, 4;
- a control system 12 having at least one computer 14;
- pumping means 16 and valve means 18 positioned in the at least one supply circuit 8 and connected to the at least one computer 14 for regulating the supply of the heating fluid from the at least one supply circuit 8 to the internal circuits 6.

The heating surfaces 2, 4 are mounted on a frame of the catalysis oven 1, generally known in the sector and not shown in the attached figures.

Each pair of surfaces 2, 4 is formed by a first surface 2, or top surface, and by a second surface 4, or bottom surface. In the context of the present description "bottom surface" is understood as meaning the surface of each pair situated at a smaller distance from the ground.

In the schematic view of Figure 1, for simpler illustration, the top surfaces 2 are grouped together and separated from the bottom surfaces 4, which are in turn grouped together. The group of top surfaces 2 is shown in a position raised with respect top the group of bottom surfaces 4.

Furthermore, the schematic view of Figure 1 shows only four top surfaces 2 and four bottom surfaces 4; however, the catalysis oven 1 may comprise a greater number of pairs of heating surfaces 2, 4 preferably at least 18 pairs, which delimit respective housings.

The heating surfaces 2, 4 are at least partially movable; for example, the bottom heating surfaces 4 may be positioned at a set height or at a variable height.

In the embodiment which comprises the bottom surfaces 4 at a set height, the casings with the mix must be raised up to said set height in order to be inserted inside the respective housing.

In the embodiment which comprises the bottom surfaces 4 able to be positioned at a variable height, the steps of inserting and extracting the casings into/from the respective housings are performed at a predefined height.

Each top surface 2 is also movable with respect to the corresponding bottom surface 4 in order to vary the dimensions of the housing and allow the insertion of the respective casing/sheath with the mix.

Each casing positioned inside the respective housing is supported by the bottom heating surface 4; the respective top heating surface 2 is moved so as to come into contact with the casing and with the mix so as to heat uniformly the mix during the catalysis and hardening step.

In accordance with an alternative embodiment, the top surfaces can be positioned at a set or variable height and the bottom surfaces associated with them are movable with respect to the top surfaces.

The transmission of the heat between the heating surfaces 2, 4 and the casings with the mix occurs mainly by means of heat conduction.

Furthermore, the heat transmission between the top surfaces and the sheaths may also take place by means of irradiation, for example in the case where the surfaces do not rest on the casings, but remain raised at a distance of a few millimetres.

The quantity of heat transmitted from the heating surfaces 2, 4 to the casings and the mix, required in order to trigger the catalysis reaction, depends on the time for which the sheath remains inside the housing and the temperature at which the surfaces 2, 4 are kept.

The heating surfaces 2, 4 are preferably made of aluminium so as to have a relatively low specific weight and high thermal conductivity.

The at least one internal circuit 6 of each heating surface 2, 4 is arranged in the manner of a suitably shaped coil over the whole area of the heating surface 2, 4 so as to heat it in a uniform manner. Preferably, the heating fluid is diathermic oil.

Conveniently, each of the heating surfaces 2, 4 comprises at least four internal circuits 6 and, for constructional reasons, may be divided into two half-surfaces 2A, 2B; 4A, 4B, each of which comprises a pair of internal circuits 6, as shown schematically in Figure 1.

In particular, in the schematic view of Figure 1, the half-surfaces 2A, 2B; 4A, 4B of each surface 2, 4 are to be regarded as arranged alongside each other. In this way, if each half-surface 2A, 2B; 4A, 4B covers half a slab, the configuration indicated above allows the whole area of the slab to be covered.

Furthermore, the coils of the internal circuits 6 are arranged preferably intersecting so that the heat is transmitted onto the surface of the slab as uniformly as possible.

In the schematic view of Figure 1, the internal circuits 6 are shown as straight broken lines for simpler illustration.

Furthermore, the internal circuits 6 of each half-surface 2A, 2B and 4A, 4B are arranged alongside each other and the input of an internal circuit 6 coincides with the output of the other internal circuit 6.

The catalysis oven 1 may comprise, for each heating surface 2, 4, a device for controlling the temperature of the heating surfaces 2, 4, connected to the at least one computer 14, the function of which will be explained below.

The temperature control device is shown in the schematic view of Figure 2 with reference to the third automatic detection means 36 described below.

The at least one circuit 8 for supplying the heating fluid is connected to at least one exchanger 10 of a boiler and comprises preferably:
- a primary supply circuit 9 connected to the at least one exchanger 10;
- at least two secondary supply circuits 11A, 11B, namely a first secondary circuit 11A and a second secondary circuit 11B, which are connected to the primary supply circuit 9 and to the internal circuits 6 of the heating surfaces 2, 4. The primary circuit 9 and the secondary circuits 11A, 11B are shown schematically in Figure 1.

The primary supply circuit 9 comprises preferably at least one pair of branches 9A, 9B, each branch 9A, 9B being connected to a respective secondary supply circuit 11A, 11B.

In particular, each branch 9A, 9B of the primary supply circuit 9, as well as each of the secondary supply circuits 11A, 11B, comprises a delivery section 13 and a return section 15. The delivery sections and the return sections are indicated respectively and overall by the reference numbers 13 and 15, whether they refer to the branches 9A, 9B of the primary supply circuit 9 or to the two secondary supply circuits 11A, 11B.

The delivery sections 13 of the branches 9A, 9B of the primary circuit 9 supply with the heating fluid the respective secondary circuits 11A, 11B by means of thermal circuit breakers 20, while the return sections 15 of the branches 9A, 9B receive the heating fluid from the secondary circuits 11A, 11B via respective thermal circuit breakers 20.

The thermal circuit breakers 20 may also be replaced with heat exchangers, without thereby departing from the scope of protection of the present invention.

Moreover, the delivery sections 13 of the secondary circuits 11A, 11B supply the heating surfaces 2, 4 with the heating fluid and the return sections 15 of the secondary circuits 11A, 11B receive the heating fluid from the heating surfaces 2, 4.

In accordance with a preferred embodiment of the invention, the first surfaces or top surfaces 2 of the pairs are connected to the first secondary circuit 11A and the second surfaces or bottom surfaces 4 of the pairs are connected to the second secondary circuit 11B, as schematically shown in Figure 1.

With this configuration it is possible:
- to have the at least one primary circuit 9 operating at a working temperature close to 190-210°C and to keep the heating surfaces 2, 4 at a temperature within the range 90-120°C.
- to keep the temperature difference of the heating fluid in the catalysis oven 1 between the delivery section 13 and the return section 15 of each secondary circuit 11A, 11B, at a value of less than 4°C despite the fact that the temperature difference of the heating fluid input into and output from the at least one exchanger 10 of the boiler, and therefore between the delivery section 13 and the return section 15 of the primary circuit 9, is close to 40°C.

The number of secondary circuits 11A, 11B may also be different from that indicated above, irrespective as to the number of heating surfaces 2, 4 provided in the oven 1 without thereby departing from the scope of protection of the present invention.

Conveniently the pumping means 16 comprise one or more electric pumps or circulators positioned in the primary supply circuit 9 and the secondary supply circuits 11A, 11B, respectively.

Furthermore, the valve means 18 may comprise:
- a pneumatic three-way valve 22 arranged between each delivery section 13 and each return section 15 of the two branches 9A, 9B of the primary supply circuit 9 and connected to the at least one computer 14 of the control system 12;
- a two-way modulating, pneumatic compensation valve 24 arranged between each delivery section 13 and each return section 15 of the two secondary circuits 11A, 11B downstream of the respective electric pump 16.

In particular, the function of the three-way pneumatic valves 22 is that of selectively regulating the flowrate of the heating fluid supplied to the secondary circuits 11A, 11B and of varying consequently the temperature of the heating surfaces 2, 4 depending on the heat flow required by the operating conditions.

For example, in the case where it is required to increase the amount of heat to be supplied to the heating surfaces 2, 4 and therefore increase the temperature thereof, the computer 14 controls the three-way valves 22 so as to increase the flow, and therefore the flowrate, of heating fluid directed towards the respective secondary circuits 11A, 11B.

On the other hand, if it is required to reduce the amount of heat supplied to the heating surfaces 2, 4 in order to reduce the temperature thereof, the computer 14 controls operation of the three-way valves 22 so that they deviate partially the heating fluid from the exchanger 10 of the boiler towards the corresponding return section 15, reducing the flow intended for the respective secondary circuits 11A, 11B.

The catalysis oven 1 may also comprise, in each secondary supply circuit 11A, 11B, at least one flowmeter, shown in the schematic view of Figure 2 with reference to the third automatic detection means 36 described below. The flowmeters are also connected to the at least one computer 14.

The selective activation of the two-way modulating pneumatic valve 24 of each secondary circuit 11A, 11B is performed depending on the flowrate detected by the respective flowmeter.

If the flowrate detected in the respective secondary circuit 11A, 11B falls below a threshold value, the corresponding modulating two-way pneumatic valves 24 are selectively opened.

For example, if the flowrate detected by the flowmeter decreases, the modulating two-way pneumatic valve 24 will open further, and vice versa.

This operating mode prevents irregular functioning of the various electric pumps of the secondary circuit when there is a variation in the flow in the internal circuits 6 of the surfaces 2, 4; in this way the electric pumps continue to operate in a regular manner, as will also be explained below.

The catalysis oven 1 may also comprise one or more heat detectors or heat chambers positioned at the output of the oven 1 in order to detect the temperature of the surfaces of the hardened slabs after their removal from the casings and/or a pressure sensor for each secondary circuit 11A, 11B designed to signal any malfunctioning of the supply circuit 8.

The heat chambers or heat detectors are shown in the schematic view of Figure 2 with reference to the four automatic detection means 36 described below.

In accordance with a peculiar aspect of the invention, the valve means 18 comprise a plurality of actuated valves 26 consisting of a number corresponding to the number of internal circuits 6 of the heating surfaces 2, 4 and connected to the at least one computer 14, as schematically shown in Figure 2, in order to regulate selectively the supply of the heating fluid to the internal circuits 6. Preferably, the actuated valves 26 are shut-off valves.

Advantageously, actuation of the valves may be performed electromechanically or pneumatically, or also in a different manner, and is controlled by the computer 14. Therefore, the actuated shut-off valves 26 are electric valves or pneumatic valves.

Each actuated shut-off valve 26 is connected to the at least one computer 14 of the control system 12 in order to intercept selectively the supply of the heating fluid from the at least one supply circuit 8 to the respective internal circuit 6.

In particular, the secondary supply circuits 11A, 11B are connected to each of the internal circuits 6 of the heating surfaces 2, 4 with the arrangement, in between, of respective actuated shut-off valves 26.

Considering that, as indicated above, each heating surface 2, 4 comprises at least four internal circuits 6 and each of these internal circuits 6 is supplied with the heating fluid by means of a respective actuated shut-off valve 26, each heating surface 2, 4 is provided with four actuated shut-off valves 26.

As a result of this arrangement it is possible to regulate in a precise and independent manner the heat flow towards each heating surface 2, 4.

In this connection, it should be emphasized that the four actuated shut-off valves 26 of each heating surface 2, 4 are activated in unison so as to regulate the heat flow of said heating surface 2, 4.

Therefore, the catalysis oven 1 of the present invention has a greater flexibility than the known catalysis ovens, since it allows the catalysis and hardening step to be carried out simultaneously and independently on a plurality of mixes which may also have mutually different characteristics.

It should also be emphasized that, if the heat flow provided to a surface, and from this surface to the slab, must be 70% of the maximum amount deliverable, the corresponding actuated valve(s) 26 must remain open for about 70% of the time needed for the catalysis reaction, namely for a duration of about 21 minutes if the catalysis time is about 30 minutes. Conveniently, the time interval for which the actuated valve must remain open or closed must be suitably distributed within the overall catalysis time; for example, the closure of the actuated valve could be programmed after the mix has reached 80°C and after the exothermic reaction has started.

The surfaces 2, 4 thus heated may also heat the adjacent surfaces to a lower temperature by means of irradiation; in order to prevent this happening the catalysis oven 1 may comprise layers of insulating material arranged between the pairs of surfaces 2, 4.

The actuated shut-off valves 26 associated with the heating surfaces 2, 4 are positioned at the input of each internal circuit 6 and are preferably two-way electric valves, or pneumatic valves, of the on/off type.

Alternatively, the actuated shut-off valves 26 may also be chosen from modulating valves, although this choice results in an increase in the complexity and costs of the catalysis oven. Therefore, each of the internal circuits 6 of the heating surfaces 2,4 or of the respective half-surfaces 2A, 2B; 4A, 4B is supplied with the heating fluid by means of a respective actuated shut-off valve 26.

The selective activation of each actuated valve 26 may also be controlled depending on the temperature of the respective heating surface 2, 4 detected by the temperature control device associated with said heating surface 2, 4 and described above.

In this way, it is possible to determine beforehand a set point or threshold temperature for each pair of heating surfaces 2, 4 to be reached before insertion of the casing with the mix inside the respective housing.

The threshold temperature is reached by regulating the flow of heating fluid to the heating surfaces 2, 4 by means of opening or closing of the actuated valves 26 controlled by the at least one computer 14.

Moreover, the set point temperature is maintained by means of a control system comprising innovative regulators based on the digital model of the catalysis oven 1, or traditional digital regulators such as PIDs (proportional-integrative-derivative controllers) to be implemented in the control system 12 of the catalysis oven 1.

The above-described flowmeters and two-way modulating valves 24 present in the secondary circuits 11A, 11B are able to compensate for the variations in flowrate due to the selective activation of the actuated shut-off valves 26 and to avoid irregular functioning of the electric pumps 16 located in the secondary circuits 11A, 11B, as already indicated above.

The control system 12, which is shown schematically in Figure 2, preferably comprises at least one first processing unit 28 with a first software module based on at least one artificial intelligence algorithm and connected to the at least one computer 14.

The control system 12 also comprises first automatic detection means 30 and second automatic detection means 32 connected to the at least one computer 14.

The first automatic detection means 30 are configured to detect automatically first values relating to the environmental operating conditions of the catalysis oven 1 and the second automatic detection means 32 are configured to detect automatically second values relating to characteristics of the slabs to be catalysed.

The first values relate preferably to the temperature of the environment and in some cases the humidity of the environment, and the second values relate preferably to the features of the slabs which are compacted and therefore to be catalysed, in particular the thickness, the actual weight, the composition of the mix, the amount and the type of resin and activators and regulators of the catalysis and the topography of the casing which encloses the slab, on which the area of contact of the top heating surface 2 on the casing depends.

These first and second values form an input for processing by the first processing unit 28.

As shown in Figure 2, at least a first controller 34 is provided, connected to the at least one computer 14 and at least to the selective components of the heating surfaces 2, 4, in particular to the actuated valves 26, for selective operation thereof.

The first and second values indicated above, which represent an input, after being collected and stored by the at least one computer 14, are intended to be processed by the first processing unit 28 in order to obtain optimized reference values of the operating parameters of the catalysis oven 1.

These optimized reference values represent an output of the processing process performed by the first processing unit 28.

The optimized reference values of the operating parameters are transmitted from the at least one computer 14 to the first controller 34 for selective control of the components of the catalysis oven 1, in particular for selective activation of the actuated valves 26 and the three-way pneumatic valves 22, and to the means for selective movement of the heating surfaces 2, 4.

The optimized reference values of the operating parameters are such that the slabs obtained following the catalysis and hardening process are substantially without defects, such as distortions or curvatures.

These operating parameters consist mainly of the time required to supply the fluid to the heating surfaces 2, 4, the temperatures and the flowrates of the heating fluids in the circuits 9, 11A, 11B and the movement time of the heating surfaces 2, 4 and are specific for different types of mixes.

Furthermore, the predefined initial values of these operating parameters may be contained beforehand in a formulation or set-up prepared by a dedicated program of the at least computer 14.

As described further below with reference to the method, the optimized reference values are used to carry out successive catalysis and hardening operations on further mixes.

The at least one computer 14 may also be configured to receive and store further information about initial conditions which may be directly entered by the operator into the at least one computer 14.

This information may also be obtained or derived/read by other systems or means, such as the production system 48, which are independent of the first detection means 30 and the second detection means 32.

These initial conditions relate mainly to characteristics of the catalysis oven, characteristics of the sheaths and characteristics of the mix components.

The further initial conditions also form, in combination with the first values and with the second values indicated above, an input for the processing process carried out by the first processing unit 28.

These initial conditions comprise preferably:
- the nature and the composition of the various starting components of the mix and in particular the type and the quantity of binder and activators and catalyst regulators and the type of granules and aggregates;
- the characteristics of the slabs to be obtained and therefore, for example, the possible presence of veining in the slabs to be formed;
- the thickness and characteristics of the sheaths or casings;
- the characteristics of the catalysis oven 1, such as the material of the heating surfaces 2, 4, the specific heat, the thermal inertia and the thermal conductivity of the heating surfaces 2, 4. Also the information relating to the initial conditions is intended to be processed by the first processing unit 28 and helps obtain the optimized reference values indicated above.

The control system 12 advantageously also comprises third means 36 for automatic detection of third values relating to operating parameters of the catalysis oven 1, also connected to the at least one computer 14 by means of the first controller 34.

As mentioned, the third automatic detection means 36 may be chosen from the group which comprises pressure gauges, thermostats, flowmeters, heat probes for detecting the temperature of the heating fluid in the circuits 9, 11A, 11B and for detecting the temperature of the heating surfaces 2, 4.

The third values detected by the first controller 34, in combination with the first values, with the second values and with the initial conditions, are transmitted to the at least one computer 14 so as to be processed by the first processing unit 28 in order to obtain the optimized reference values.

Therefore the third values also form an input for the processing process by the first processing unit 28 in combination with the first and second values and with the initial conditions.

Also provided are fourth means 38 for the automatic detection of fourth values relating to dimensions and characteristics of the slabs following the initial hardening of the mix and/or fifth means 40 for the automatic detection of fifth values relating to the dimensions and characteristics of the slabs following cooling thereof, since any distortions due to imperfections during the catalysis step are evident only after cooling.

Therefore, the fourth automatic detection means 38 are located at the output of the catalysis oven 1 and the fifth automatic detection means 40 are located at the output of the slab cooling device.

The fourth values relate preferably to the following:
- the temperature of the faces of the hardened slabs following the catalysis reaction;
- the hardness/consistency of the hardened slabs following the catalysis reaction;
- the thickness of the hardened slabs following the catalysis reaction;
- the topography of the surface of the slabs.

In this connection, the fourth detection means 38 may comprise heat chambers, heat sensors, hardness gauges or thickness gauges, as mentioned above.

The fifth values relate preferably to the following:
- the thickness of the slabs following the cooling step;
- the flatness of the surfaces of the slabs following the cooling step.

For this purpose, the fifth detection means 40 may comprises thickness and flatness gauges which are located at the output of the cooling tower.

Advantageously, the fourth automatic detection means 38 and the fifth automatic detection means 40 are connected at least indirectly to an additional computer 42 of the control system 12, preferably by means of a second controller 44.

The additional computer 42 is connected to at least one second processing unit 46 having a second software module based on at least one artificial intelligence algorithm.

The algorithm of the second processing unit 46 is configured to process and/or classify the fourth and fifth values and to implement the first software module of the first processing unit 28 based on these values.

In particular, the algorithm of the second processing unit 46 is configured to collect, catalogue and process the fourth values and the fifth values as a whole, thus performing so-called "data fusion".

Therefore, the fourth and fifth values also form an input for the processing process intended to obtain optimized reference values of the operating parameters as output of the processing process.

Furthermore, the additional computer 42 may be configured to receive and store further information relating to the final conditions of the catalysis process, namely the final characteristics of the slabs.

Said further information may be directly entered by the operator into the additional computer 42 or may be obtained or derived/read by other systems or means independent of the fourth detection means 38 and the fifth detection means 40.

The at least one algorithm of the software modules of the first and second processing units 28, 46 may be of the machine learning, deep learning or reinforcement learning type or also a combination of the three types.

In accordance with an alternative embodiment, the first processing unit 28 and the second processing unit 46 may also be physically integrated into one of the computers 14, 42 instead of being separate as indicated above.

The first controller 34 and the second controller 44 are preferably PLCs (programmable logic controllers) or PACs (programmable automation controllers).

Owing to the processing performed by the second processing unit 46 it is possible to obtain the most accurate possible evaluation as to the degree of quality of the slabs obtained, in view of the presence of any defects such as deformations and distortions.

With regard to the above it should be noted that:
- the first automatic detection means 30, second automatic detection means 32 and third automatic detection means 36 as well as the systems for detecting the initial conditions are designed to control the operation of the oven;
- the fourth automatic detection means 38 and the fifth detection means 40 as well as the systems for detecting the final conditions are designed to control the quality of the slabs obtained following the catalysis and hardening process and the cooling process, respectively. The possibility is also envisaged of the operator being able to intervene and support the control system 12, with particular reference to the second processing unit 46.

In this connection, the operator may visually detect the presence of defects on the slabs obtained following hardening or cooling of the slabs, in particular in the case where detection is particularly complex and/or costly to perform by means of the detection means.

Then the operator may enter the information detected into the second processing unit 46 so that said information is managed and processed in the same way as the values detected by the detection means.

The present invention also relates to a method for controlling operation of the catalysis oven 1 of the type described above for the production of slabs of agglomerated material.

The method comprises the following steps:
- carrying out several test processes using the catalysis oven 1 in order to perform the catalysis and hardening of mixes and obtain slabs using predetermined operating conditions;
- automatically detecting and collecting at least one series of values relating to the environmental operating conditions of the catalysis oven 1 and/or characteristics of the casings and/or of the compacted slabs to be catalysed, detection and collection of the characteristics of the slabs to be obtained and/or operating characteristics and parameters of the catalysis oven 1 and/or characteristics of the casings and/or characteristics of the components forming the mix and automatic detection and collection of the dimensions and characteristics of the slabs following hardening and cooling of the slabs;
- analysis and processing of said at least one series of values in order to derive and obtain optimized reference values of the operating parameters of the catalysis oven 1;
- execution of one or more processes for catalysis and hardening of the mix in order to form the slabs using the catalysis oven 1 based on the optimized reference values obtained.

The analysing and processing step is performed by means of the training of at least one first software module based on at least one artificial intelligence algorithm.

Conveniently, the step of detecting and collecting the at least one series of values is performed by means of one or more of the first detection means 30, the second detection means 32, the third detection means 36, the fourth detection means 38 and the fifth detection means 40 described above.

Furthermore, the method uses a second software module based on at least one artificial intelligence algorithm and configured to implement the first software module. The second software module coincides with the module indicated above with reference to the second processing unit 46 of the control system 12.

As mentioned with reference to the catalysis oven 1, the values normally detected by the detection means may also be detected and manually entered by the operator, in particular into the second processing unit 46.

Furthermore, further information regarding initial conditions of the catalysis process and/or final conditions of the slabs, of the type indicated above, may be stored in the computers 14, 42.

As already mentioned, the values detected and collected in the corresponding step constitute an input for the subsequent analysis and processing step.

Furthermore the processing step is performed by means of the first processing unit 28 and/or the second processing unit 46 of the control unit 12 described above.

As already mentioned, the optimized reference values obtained with the analysis and processing step constitute an output of the processing process.

The first step involves the execution of several test processes and may be repeated several times before forming the finished slabs, so as to detect and collect a large number of values which allow efficient training of the control system 12 using the methods described above, resulting in particularly efficient control and management of the catalysis oven 1.

From the above description it is now clear how the catalysis oven and the method according to the present invention are able to achieve advantageously the predefined objects.

In particular, the combined use of a series of actuated shut-off valves consisting of a number corresponding to the internal circuits of the heating surfaces, and the control system described above, allow a predefined heat flow to be established and maintained for each surface depending on the characteristics of the mix used, the characteristics of the casings, the environmental conditions and the slabs to be obtained.

This advantage means that the catalysis oven according to the present invention is particularly flexible and is able to limit the formation of defects in the slabs, such as deformations or curvatures.

## Claims

1. Catalysis oven (1) for hardening an agglomerated mix of stone or stone-like material with an organic binder, compacted inside casings or sheaths and intended to form slabs, comprising:
- a series of pairs of heating surfaces (2, 4) which delimit respective housings for the casings containing the mix, each of said surfaces (2, 4) comprising at least one internal circuit (6) for a heating fluid;
- at least one circuit (8) for supplying the heating fluid, said at least one supply circuit (8) being connected to the internal circuits (6) of said heating surfaces (2, 4);
- a control system (12) having at least one computer (14);
- pumping means (16) and valve means (18) positioned in said at least one supply circuit (8) and connected to said at least one computer (14) so as to regulate the supply of the heating fluid;
**characterized in that** said valve means (18) comprise a plurality of actuated valves (26) consisting of a number corresponding to the number of internal circuits (6) of said heating surfaces (2, 4), said actuated valves (26) being connected to said at least one computer (14) so as to regulate selectively the supply of the heating fluid to said internal circuits (6).

2. Catalysis oven (1) according to the preceding claim, **characterized in that** said actuated valves (26) are shut-off valves.

3. Catalysis oven (1) according to the preceding claim, **characterized in that** said actuated shut-off valves (26) are electric valves or pneumatic valves, preferably of the on/off two-way type, or modulating valves.

4. Catalysis oven (1) according to any one of the preceding claims, **characterized in that** each of said heating surfaces (2, 4) comprises at least four internal circuits (6), each heating surface (2, 4) being divided into two half-surfaces (2A, 2B; 4A, 4B) so as to cover the area of the slab, each of said half-surfaces (2A, 2B; 4A, 4B) comprising a pair of internal circuits (6).

5. Catalysis oven (1) according to any one of the preceding claims, **characterized in that** said at least one supply circuit (8) comprises a primary supply circuit (9) and at least two secondary supply circuits (11A, 11B) connected to said primary supply circuit (9) and to the internal circuits (6) of said heating surfaces (2, 4) with the arrangement of said actuated valves (26) in between.

6. Catalysis oven (1) according to Claim 5, **characterized in that** said primary supply circuit (9) comprises at least one pair of branches (9A, 9B), each branch (9A, 9B) of said primary supply circuit (9) being connected to a respective secondary supply circuit (11A, 11B) and comprising a delivery section (13) and a return section (15), said valve means (18) comprising a three-way pneumatic valve (22) arranged between each delivery section (13) and the respective return section (15) of each branch (9A, 9B) of said primary supply circuit (9).

7. Catalysis oven (1) according to any one of Claims 5-6, **characterized in that** each of said secondary circuits (11A, 11B) comprises a delivery section (13) and a return section (15), said valve means comprising a two-way modulating pneumatic valve (24) arranged between each delivery section (13) and the respective return section (15) of each of said secondary circuits (11A, 11B).

8. Catalysis oven (1) according to any one of the preceding claims, **characterized in that** said control system (12) comprises at least one first processing unit (28) with a first software module based on at least one artificial intelligence algorithm and connected to said at least one computer (14), said at least one algorithm being of the machine learning, deep learning and reinforcement learning type or also a combination of these types.

9. Catalysis oven (1) according to the preceding claim, **characterized in that** said control system (12) comprises first automatic detection means (30) and second automatic detection means (32) connected to said at least one computer (14), said first detection means (30) being configured for the detection of first values relating to environmental operating conditions of the catalysis oven (1) and said second detection means (32) being configured to detect second values relating to characteristics of the casings and/or characteristics of the mix, said first values and said second values being processed by said first processing unit (28) in order to obtain optimized reference values of the operating parameters of the catalysis oven (1).

10. Catalysis oven (1) according to the preceding claim, **characterized in that** said control system (12) comprises at least one first controller (34) connected to the at least one computer (14) and to at least the heating surfaces (2, 4) and to the actuated valves (26) for selective operation thereof, said at least one computer (14) being able to transmit the optimized reference values to said first controller (34).

11. Catalysis oven (1) according to the preceding claim, **characterized in that** said control system (12) comprises third means (36) for the automatic detection of third values relating to operating parameters of the catalysis oven (1), said third detection means (36) being connected to said at least one computer (14) via said first controller (34), and **in that** said control system (12) comprises fourth means (38) for automatic detection of fourth values relating to dimensions and characteristics of the slabs following the mix hardening step, and/or fifth means (40) for automatic detection of fifth values relating to dimensions and characteristics of the slabs following cooling thereof.

12. Catalysis oven (1) according to the preceding claim, **characterized in that** said fourth detection means (38) and said fifth detection means (40) are connected to an additional computer (42) of said control system (12) via a second controller (44).

13. Catalysis oven (1) according to the preceding claim, **characterized in that** said additional computer (42) is connected to at least one second processing unit (46) with a second software module based on at least one artificial intelligence algorithm of the machine learning, deep learning and reinforcement learning type or a combination of said types, said second software module being configured to process and/or classify said fourth and fifth values and to implement said first software module of said first processing unit (28) based on said processed fourth values and fifth values.

14. Method for controlling the operation of a catalysis oven (1), of the type according to one or more of Claims 1-13, for the production of slabs of agglomerated stone or stone-like material using a mix with an organic binder, comprising the following steps:
- carrying out several test processes using the catalysis oven (1) in order to perform the catalysis and hardening of the mix and obtain slabs using predetermined operating conditions;
- detection and automatic collection of at least one series of values relating to the environmental operating conditions of the catalysis oven (1) and/or characteristics of the sheaths or casings and/or of the mix and/or of the slabs to be obtained and/or operating parameters of the catalysis oven (1) and/or dimensions and characteristics of the slabs following the hardening process and/or dimensions and characteristics of the slabs following cooling of the slabs;
- analysis and processing of said at least one series of values in order to obtain optimized reference values of the operating parameters of the catalysis oven (1);
- execution of one or more processes of catalysis and hardening of the mix in order to obtain slabs using the catalysis oven (1) based on the said optimized reference values;
wherein said analysis and processing step is performed by means of training of at least one first software module based on at least one artificial intelligence algorithm of the machine learning, deep learning and reinforcement learning type or a combination of these types.

15. Method according to the preceding claim, **characterized in that** the method involves the use of a second software module based on at least one artificial intelligence algorithm and configured to implement said first software module.

## Patentansprüche

1. Katalyseofen (1) zum Aushärten einer agglomerierten Mischung aus Stein oder steinartigem Material mit einem organischen Bindemittel, die in Gehäusen oder Hüllen verdichtet ist und zur Bildung von Platten bestimmt ist, umfassend:
- eine Reihe von Paaren von Heizflächen (2, 4), die jeweilige Gehäuse für die die Mischung enthaltenden Hüllen begrenzen, wobei jede der Flächen (2, 4) mindestens einen internen Kreislauf (6) für ein Heizfluid umfasst;
- mindestens einen Kreislauf (8) zum Zuführen des Heizfluids, wobei der mindestens eine Zufuhrkreislauf (8) mit den internen Kreisläufen (6) der Heizflächen (2, 4) verbunden ist;
- ein Steuersystem (12) mit mindestens einem Computer (14);
- eine Pumpeinrichtung (16) und eine Ventileinrichtung (18), die in dem mindestens einen Zufuhrkreislauf (8) angeordnet und mit dem mindestens einen Computer (14) verbunden sind, um die Zufuhr des Heizfluids zu regeln;
**dadurch gekennzeichnet, dass** das Ventilelement (18) eine Mehrzahl von angesteuerten Ventilen (26) umfasst, deren Anzahl der Anzahl der internen Kreisläufe (6) der Heizflächen (2, 4) entspricht, wobei die angesteuerten Ventile (26) mit dem mindestens einen Computer (14) verbunden sind, um die Zufuhr des Heizfluids zu den internen Kreisläufen (6) selektiv zu regeln.

2. Katalyseofen (1) nach dem vorstehenden Anspruch, **dadurch gekennzeichnet, dass** die angesteuerten Ventile (26) Absperrventile sind.

3. Katalyseofen (1) nach dem vorstehenden Anspruch, **dadurch gekennzeichnet, dass** die angesteuerten Absperrventile (26) elektrische Ventile oder pneumatische Ventile, vorzugsweise vom Typ Ein/Aus-Zweiwegeventil, oder Modulationsventile sind.

4. Katalyseofen (1) nach einem der vorstehenden Ansprüche, **dadurch gekennzeichnet, dass** jede der Heizflächen (2, 4) mindestens vier interne Kreisläufe (6) umfasst, wobei jede Heizfläche (2, 4) in zwei Halbflächen (2A, 2B; 4A, 4B) unterteilt ist, um die Fläche der Platte zu bedecken, wobei jede der Halbflächen (2A, 2B; 4A, 4B) ein Paar interner Kreisläufe (6) umfasst.

5. Katalyseofen (1) nach einem der vorstehenden Ansprüche, **dadurch gekennzeichnet, dass** der mindestens eine Zufuhrkreislauf (8) einen Primärzufuhrkreislauf (9) und mindestens zwei Sekundärzufuhrkreisläufe (11A, 11B) umfasst, die mit dem Primärzufuhrkreislauf (9) und den internen Kreisläufen (6) der Heizflächen (2, 4) verbunden sind, wobei die angesteuerten Ventile (26) dazwischen angeordnet sind.

6. Katalyseofen (1) nach Anspruch 5, **dadurch gekennzeichnet, dass** der Primärzufuhrkreislauf (9) mindestens ein Paar von Zweigen (9A, 9B) umfasst, wobei jeder Zweig (9A, 9B) des Primärzufuhrkreislaufs (9) mit einem entsprechenden Sekundärzufuhrkreislauf (11A, 11B) verbunden ist und einen Zuleitungsabschnitt (13) und einen Rücklaufabschnitt (15) umfasst, wobei das Ventilelement (18) ein Dreiwege-Pneumatikventil (22) umfasst, das zwischen jedem Zuleitungsabschnitt (13) und dem jeweiligen Rücklaufabschnitt (15) jedes Zweigs (9A, 9B) des Primärzufuhrkreislaufs (9) angeordnet ist.

7. Katalyseofen (1) nach einem der Ansprüche 5 bis 6, **dadurch gekennzeichnet, dass** jeder der Sekundärkreisläufe (11A, 11B) einen Zuleitungsabschnitt (13) und einen Rücklaufabschnitt (15) umfasst, wobei das Ventilelement ein Zweiwege-Modulations-Pneumatikventil (24) umfasst, das zwischen jedem Zuleitungsabschnitt (13) und dem jeweiligen Rücklaufabschnitt (15) jedes der Sekundärkreisläufe (11A, 11B) angeordnet ist.

8. Katalyseofen (1) nach einem der vorstehenden Ansprüche, **dadurch gekennzeichnet, dass** das Steuersystem (12) mindestens eine erste Verarbeitungseinheit (28) mit einem ersten Softwaremodul umfasst, das auf mindestens einem Algorithmus künstlicher Intelligenz basiert und mit dem mindestens einen Computer (14) verbunden ist, wobei der mindestens eine Algorithmus von der Art maschinellen Lernens, Deep Learning und verstärkenden Lernens oder auch eine Kombination dieser Arten ist.

9. Katalyseofen (1) nach einem der vorstehenden Ansprüche, **dadurch gekennzeichnet, dass** das Steuersystem (12) eine erste automatische Erfassungseinrichtung (30) und eine zweite automatische Erfassungseinrichtung (32) umfasst, die mit dem mindestens einen Computer (14) verbunden sind, wobei die erste Erfassungseinrichtung (30) zur Erfassung erster Werte in Bezug auf Umgebungsbetriebsbedingungen des Katalyseofens (1) und die zweite Erfassungseinrichtung (32) zur Erfassung zweiter Werte in Bezug auf Eigenschaften der Gehäuse und/oder Eigenschaften der Mischung ausgebildet ist, wobei die ersten Werte und die zweiten Werte von der ersten Verarbeitungseinheit (28) verarbeitet werden, um optimierte Referenzwerte der Betriebsparameter des Katalyseofens (1) zu erhalten.

10. Katalyseofen (1) nach dem vorstehenden Anspruch, **dadurch gekennzeichnet, dass** das Steuersystem (12) mindestens eine erste Steuerung (34) umfasst, die mit dem mindestens einen Computer (14) und mindestens den Heizflächen (2, 4) und mit den angesteuerten Ventilen (26) zu deren selektiver Betätigung verbunden ist, wobei der mindestens ein Computer (14) die optimierten Referenzwerte an die erste Steuerung (34) übermitteln kann.

11. Katalyseofen (1) nach dem vorstehenden Anspruch, **dadurch gekennzeichnet, dass** das Steuersystem (12) eine dritte Einrichtung (36) zur automatischen Erfassung dritter Werte umfasst, die sich auf Betriebsparameter des Katalyseofens (1) beziehen, wobei die dritte Erfassungseinrichtung (36) über die erste Steuerung (34) mit dem mindestens einen Computer (14) verbunden ist, und dass das Steuersystem (12) eine vierte Einrichtung (38) zur automatischen Erfassung von vierten Werten umfasst, die sich auf Abmessungen und Eigenschaften der Platten nach dem Mischungsverfestigungsschritt beziehen, und/oder eine fünfte Einrichtung (40) zur automatischen Erfassung von fünften Werten, die sich auf Abmessungen und Eigenschaften der Platten nach deren Abkühlung beziehen.

12. Katalyseofen (1) nach dem vorstehenden Anspruch, **dadurch gekennzeichnet, dass** die vierte Erfassungseinrichtung (38) und die fünfte Erfassungseinrichtung (40) über eine zweite Steuerung (44) mit einem zusätzlichen Computer (42) des Steuersystems (12) verbunden sind.

13. Katalyseofen (1) nach dem vorstehenden Anspruch, **dadurch gekennzeichnet, dass** der zusätzliche Computer (42) mit mindestens einer zweiten Verarbeitungseinheit (46) mit einem zweiten Softwaremodul verbunden ist, das auf mindestens einem Algorithmus künstlicher Intelligenz von der Art maschinellen Lernens, Deep Learning und verstärkenden Lernens oder einer Kombination dieser Arten basiert, wobei das zweite Softwaremodul dazu ausgebildet ist, die vierten und fünften Werte zu verarbeiten und/oder zu klassifizieren und das erste Softwaremodul der ersten Verarbeitungseinheit (28) basierend auf den verarbeiteten vierten Werten und fünften Werten zu implementieren.

14. Verfahren zur Steuerung des Betriebs eines Katalyseofens (1) der Art gemäß einem oder mehreren der Ansprüche 1 bis 13 zur Herstellung von Platten aus agglomeriertem Stein oder steinartigem Material unter Verwendung einer Mischung mit einem organischen Bindemittel, umfassend die folgenden Schritte:
- Durchführen mehrerer Testprozesse unter Verwendung des Katalyseofens (1), um die Katalyse und Aushärtung der Mischung durchzuführen und Platten unter Verwendung festgelegter Betriebsbedingungen zu erhalten;
- Erfassen und automatisches Sammeln mindestens einer Reihe von Werten, die sich auf die Umgebungsbetriebsbedingungen des Katalyseofens (1) beziehen und/oder Merkmale der Hüllen oder Gehäuse und/oder der Mischung und/oder der zu erhaltenden Platten und/oder Betriebsparameter des Katalyseofens (1) und/oder Abmessungen und Merkmalen der Platten nach dem Aushärtungsprozess und/oder Abmessungen und Merkmalen der Platten nach dem Abkühlen der Platten;
- Analyse und Verarbeiten der mindestens einen Reihe von Werten, um optimierte Referenzwerte der Betriebsparameter des Katalyseofens (1) zu erhalten;
- Ausführen eines oder mehrerer Katalyse- und Härtungsprozesse der Mischung, um unter Verwendung des Katalyseofens (1) basierend auf den optimierten Referenzwerten Platten zu erhalten;
wobei der Analyse- und Verarbeitungsschritt mittels des Trainings mindestens eines ersten Softwaremoduls basierend auf mindestens einem Algorithmus künstlicher Intelligenz von der Art maschinellen Lernens, Deep Learning und verstärkenden Lernens oder einer Kombination dieser Arten durchgeführt wird.

15. Verfahren nach dem vorstehenden Anspruch, **dadurch gekennzeichnet, dass** das Verfahren die Verwendung eines zweiten Softwaremoduls umfasst, das auf mindestens einem Algorithmus künstlicher Intelligenz basiert und zur Implementierung des ersten Softwaremoduls ausgebildet ist.

## Revendications

1. Four de catalyse (1) pour durcir un mélange aggloméré de pierre ou de matériau de type pierre avec un liant organique, compacté à l'intérieur d'enveloppes ou de gaines et destiné à former des dalles, comprenant:
- une série de paires de surfaces chauffantes (2, 4) qui délimitent des logements respectifs pour les enveloppes contenant le mélange, chacune desdites surfaces (2, 4) comprenant au moins un circuit interne (6) pour un fluide chauffant;
- au moins un circuit (8) pour l'alimentation en fluide chauffant, ledit au moins un circuit d'alimentation (8) étant relié aux circuits internes (6) desdites surfaces chauffantes (2, 4);
- un système de commande (12) ayant au moins un ordinateur (14);
- des moyens de pompage (16) et des moyens de vanne (18) positionnés dans ledit au moins un circuit d'alimentation (8) et reliés audit au moins un ordinateur (14) de manière à réguler l'alimentation en fluide chauffant;
**caractérisé en ce que** lesdits moyens de vanne (18) comprennent une pluralité de vannes actionnées (26) dont le nombre correspond au nombre de circuits internes (6) desdites surfaces chauffantes (2, 4), lesdites vannes actionnées (26) étant reliées audit au moins un ordinateur (14) de manière à réguler sélectivement l'alimentation en fluide chauffant desdits circuits internes (6).

2. Four de catalyse (1) selon la revendication précédente, **caractérisé en ce que** lesdites vannes actionnées (26) sont des vannes d'arrêt.

3. Four de catalyse (1) selon la revendication précédente, **caractérisé en ce que** lesdites vannes d'arrêt actionnées (26) sont des vannes électriques ou des vannes pneumatiques, de préférence du type à deux voies marche/arrêt, ou des vannes modulantes.

4. Four de catalyse (1) selon l'une quelconque des revendications précédentes, **caractérisé en ce que** chacune desdites surfaces chauffantes (2, 4) comprend au moins quatre circuits internes (6), chaque surface chauffante (2, 4) étant divisée en deux demi-surfaces (2A, 2B ; 4A, 4B) de manière à couvrir la zone de la dalle, chacune desdites demi-surfaces (2A, 2B ; 4A, 4B) comprenant une paire de circuits internes (6).

5. Four de catalyse (1) selon l'une quelconque des revendications précédentes, **caractérisé en ce que** ledit au moins un circuit d'alimentation (8) comprend un circuit d'alimentation primaire (9) et au moins deux circuits d'alimentation secondaires (11A, 11B) reliés audit circuit d'alimentation primaire (9) et aux circuits internes (6) desdites surfaces chauffantes (2, 4), lesdites vannes actionnées (26) étant agencées entre ceux-ci.

6. Four de catalyse (1) selon la revendication 5, **caractérisé en ce que** ledit circuit d'alimentation primaire (9) comprend au moins une paire de branches (9A, 9B), chaque branche (9A, 9B) dudit circuit d'alimentation primaire (9) étant reliée à un circuit d'alimentation secondaire respectif (11A, 11B) et comprenant une section de distribution (13) et une section de retour (15), lesdits moyens de vanne (18) comprenant une vanne pneumatique à trois voies (22) agencée entre chaque section de distribution (13) et la section de retour respective (15) de chaque branche (9A, 9B) dudit circuit d'alimentation primaire (9).

7. Four de catalyse (1) selon l'une quelconque des revendications 5 à 6, **caractérisé en ce que** chacun desdits circuits d'alimentation secondaires (11A, 11B) comprend une section de distribution (13) et une section de retour (15), lesdits moyens de vanne comprenant une vanne pneumatique modulante à deux voies (24) agencée entre chaque section de distribution (13) et la section de retour respective (15) de chacun desdits circuits d'alimentation secondaires (11A, 11B).

8. Four de catalyse (1) selon l'une quelconque des revendications précédentes, **caractérisé en ce que** ledit système de commande (12) comprend au moins une première unité de traitement (28) avec un premier module logiciel basé sur au moins un algorithme d'intelligence artificielle et connecté audit au moins un ordinateur (14), ledit au moins un algorithme étant du type apprentissage automatique, apprentissage profond et apprentissage par renforcement, ou également une combinaison de ces types.

9. Four de catalyse (1) selon la revendication précédente, **caractérisé en ce que** ledit système de commande (12) comprend des premiers moyens de détection automatique (30) et des deuxièmes moyens de détection automatique (32) connectés audit au moins un ordinateur (14), lesdits premiers moyens de détection (30) étant configurés pour la détection de premières valeurs relatives aux conditions de fonctionnement environnementales du four de catalyse (1) et lesdits deuxièmes moyens de détection (32) étant configurés pour détecter des deuxièmes valeurs relatives aux caractéristiques des enveloppes et/ou aux caractéristiques du mélange, lesdites premières valeurs et lesdites deuxièmes valeurs étant traitées par ladite première unité de traitement (28) afin d'obtenir des valeurs de référence optimisées des paramètres de fonctionnement du four de catalyse (1).

10. Four de catalyse (1) selon la revendication précédente, **caractérisé en ce que** ledit système de commande (12) comprend au moins un premier contrôleur (34) connecté à l'au moins un ordinateur (14) et au moins aux surfaces chauffantes (2, 4) et aux vannes actionnées (26) en vue de leur fonctionnement sélectif, ledit au moins un ordinateur (14) étant apte à transmettre les valeurs de référence optimisées audit premier contrôleur (34).

11. Four de catalyse (1) selon la revendication précédente, **caractérisé en ce que** ledit système de commande (12) comprend des troisièmes moyens (36) pour la détection automatique de troisièmes valeurs relatives aux paramètres de fonctionnement du four de catalyse (1), lesdits troisièmes moyens de détection (36) étant connectés audit au moins un ordinateur (14) par l'intermédiaire dudit premier contrôleur (34), et **en ce que** ledit système de commande (12) comprend des quatrièmes moyens (38) pour la détection automatique de quatrièmes valeurs relatives aux dimensions et aux caractéristiques des dalles après l'étape de durcissement du mélange, et/ou des cinquièmes moyens (40) pour la détection automatique de cinquièmes valeurs relatives aux dimensions et aux caractéristiques des dalles après leur refroidissement.

12. Four de catalyse (1) selon la revendication précédente, **caractérisé en ce que** lesdits quatrièmes moyens de détection (38) et lesdits cinquièmes moyens de détection (40) sont connectés à un ordinateur supplémentaire (42) dudit système de commande (12) par l'intermédiaire d'un deuxième contrôleur (44).

13. Four de catalyse (1) selon la revendication précédente, **caractérisé en ce que** ledit ordinateur supplémentaire (42) est connecté à au moins une deuxième unité de traitement (46) avec un deuxième module logiciel basé sur au moins un algorithme d'intelligence artificielle du type apprentissage automatique, apprentissage profond et apprentissage par renforcement, ou une combinaison de ces types, ledit deuxième module logiciel étant configuré pour traiter et/ou classer lesdites quatrièmes et cinquièmes valeurs et pour mettre en œuvre ledit premier module logiciel de ladite première unité de traitement (28) sur la base desdites quatrièmes et cinquièmes valeurs traitées.

14. Procédé pour commander le fonctionnement d'un four de catalyse (1), du type selon l'une quelconque ou plusieurs des revendications 1 à 13, en vue de la production de dalles de pierre agglomérée, ou de matériau de type pierre, à l'aide d'un mélange avec un liant organique, comprenant les étapes suivantes comprenant le fait de:
- réaliser plusieurs processus d'essai à l'aide du four de catalyse (1) afin d'effectuer la catalyse et le durcissement du mélange et d'obtenir des dalles dans des conditions de fonctionnement prédéterminées;
- détecter et collecter automatiquement au moins une série de valeurs relatives aux conditions de fonctionnement environnementales du four de catalyse (1) et/ou des caractéristiques des gaines ou des enveloppes et/ou du mélange et/ou des dalles à obtenir et/ou des paramètres de fonctionnement du four de catalyse (1) et/ou des dimensions et caractéristiques des dalles après le processus de durcissement et/ou des dimensions et caractéristiques des dalles après le refroidissement des dalles ;
- analyser et traiter ladite au moins une série de valeurs afin d'obtenir des valeurs de référence optimisées des paramètres de fonctionnement du four de catalyse (1);
- exécuter un ou plusieurs processus de catalyse et de durcissement du mélange afin d'obtenir des dalles à l'aide du four de catalyse (1) sur la base desdites valeurs de référence optimisées;
dans lequel ladite étape d'analyse et de traitement est effectuée au moyen de l'entraînement d'au moins un premier module logiciel basé sur au moins un algorithme d'intelligence artificielle du type apprentissage automatique, apprentissage profond et apprentissage par renforcement, ou une combinaison de ces types.

15. Procédé selon la revendication précédente, **caractérisé en ce que** le procédé implique l'utilisation d'un deuxième module logiciel basé sur au moins un algorithme d'intelligence artificielle et configuré pour mettre en œuvre ledit premier module logiciel.
